# EUROPÄISCHE PATENTSCHRIFT

(11) **EP 4 178 474 B1**
(45) Veröffentlichungstag und Bekanntmachung des Hinweises auf die Patenterteilung: **10.09.2025**
(21) Anmeldenummer: 21749107.5
(22) Anmeldetag: 09.07.2021
(51) Int. Cl.: A61B 17/70, A61B 17/84, A61B 17/86

(54) **KLINGENARTIGE OSTEOSYNTHESEVORRICHTUNG**
BLADE-LIKE OSTEOSYNTHETIC DEVICE
DISPOSITIF D'OSTÉOSYNTHÈSE AYANT L'APPARENCE D'UNE LAME

(30) Priorität: 11.07.2020 DE 102020004184
(43) Veröffentlichungstag der Anmeldung: 17.05.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/069206
(87) Internationale Veröffentlichungsnummer: WO 2022/013109

(56) Entgegenhaltungen:
- WO-A1-02/45606
- WO-A1-2020/160722
- US-A1- 2017 119 447

## Beschreibung

### Stand der Technik

Klingenartige Implantate werden insbesondere dann eingesetzt, wenn bei geschwächter Knochenstruktur eine Schraube zu wenig Halt bietet oder sich die Knochenfragmente bei Verwendung einer Schraube verdrehen würden. Dies ist insbesondere bei osteoporotischem Knochengewebe der Fall. Ein klingenartiges Implantat besitzt im Vergleich zu einer Knochenschraube eine größere Oberfläche und kann dadurch eine höhere Stabilität hauptsächlich gegenüber Biegebelastungen erwirken. Allerdings werden Auszugskräfte nur ansatzweise von Klingen toleriert. Hier sind Knochenschrauben stets im Vorteil.

Strukturell geschwächte Knochenstrukturen sind besonders häufig in der Wirbelsäule symptomatisch. Eine knöcherne Struktur mit der höchsten Tragfähigkeit ist der kortikale Pedikel. Deshalb ist es erstrebenswert ein klingenartiges Implantat für den Einsatz an der Wirbelsäule vorzusehen, welches sich mit der großen Oberfläche im spongiösen Wirbelkörperbereich abstützt und zusätzlich im oder am kortikalen Pedikel verankern lässt.

US9125696B2 zeigt eine Kombination aus einer Klinge mit einer Knochenschraube, die die mechanischen Vorteile einer Klinge und einer Knochenschraube miteinander vereint. Das in US9125696B2 vorgestellte Implantat ist für die allgemeine Orthopädie konzipiert und nicht für das Einbringen in den Pedikel geeignet. Dem Stand der Technik sind Pedikelschrauben (US6063090A) zu entnehmen, die eine polyaxiale Einstellung des Implantatkopfes gegenüber dem Knochenanker erlauben. Hierfür ist mindestens ein Kugelsegment am Knochenanker-Kopf notwendig, was bei der vorgestellten Lösung aus US9125696B2 nicht ersichtlich ist. Des Weiteren ist die Klinge geradlinig ohne Verdrehung der Flügelflächen gestaltet, so dass ein Einsatz im Pedikel keinen Sinn macht. In finaler Position im Pedikel würde der distale Bereich der Klinge im Wirbelkörper hochkant zur Lastrichtung stehen. Eine Verdrehung oder Verschränkung der Klingenflächen ist deshalb eine biomechanische Voraussetzung, damit Biegebelastungen am distalen Implantatsbereich innerhalb des Wirbelkörpers aufgenommen werden können.

WO0245606A1 zeigt ein verdreht-klingenartiges Implantat für die Behandlung der Wirbelsäule. In einer Ausgestaltungsform wird die Kombination mit einer sogenannten Transportschraube vorgestellt, welche in einem Fenster innerhalb des Klingenbereichs vorgesehen ist. Der Anmeldung fehlen wesentliche Details in der Ausgestaltungsform mit Transportschraube, die zur Realisierung eines funktionierenden Aufbaus notwendig wären. Strukturelle Aspekte, die zur Sicherstellung einer ausreichenden Dauerfestigkeit gegenüber wiederkehrenden Biegebelastungen, wurden ebenfalls nicht berücksichtigt. Ein Fenster für die Transportschraube schwächt die strukturelle Integrität der Klinge, so dass von einer stark reduzierten Dauerfestigkeit bei diesem Design auszugehen ist. Ebenso zeigt die WO0245606A1 eine Ausgestaltungsform mit einem Kugelsegment im Kopfbereich für den Einsatz eines polyaxialen Implantat-Kopfes. Allerdings ist die technische Ausgestaltung einer Version mit Kugel-Kopf-segment und einer funktionierenden Transportschraube nicht gezeigt, da die vorgesehenen Verbindungseinheiten von Kugel und Transportschraube sich gegenseitig ausschließen und einen gemeinsamen Aufbau behindern. Hier soll die erfindungsgemäße Osteosynthesevorrichtung Abhilfe schaffen. Die US 2017/119447 A1 offenbart eine Pedikelankervorrichtung, die so ausgestattet ist, dass sie wie eine Pedikelschraube verwendet werden kann, d. h. von einer dorsalen Richtung (jedoch im allgemeinen in einem Winkel zur Sagittalebene, leicht nach innen zur Sagittalebene hin) durch den Pedikel in den Wirbel implantiert wird, so dass ein distaler Abschnitt des Gerätes in den Wirbelkörper hineinragt. Die Pedikelverankerungsvorrichtung umfasst einen Vorrichtungskörper mit einem Kopfabschnitt, einem Schaftabschnitt und einer Längsbohrung, die sich von einem proximalen Ende des Vorrichtungskörpers erstreckt und ein Loch oder mehrere Löcher von der Längsbohrung nach außen aufweist, z B. radial nach außen.

### Darstellung der Erfindung

Die Aufgabe wird durch eine Vorrichtung nach Anspruch 1 gelöst. Weitere, die Erfindung ausgestaltende Merkmale sind in den abhängigen Ansprüchen enthalten.

In der hier vorgestellten Erfindung, wird dies dadurch gelöst, dass alle biomechanisch wichtigen Aspekte miteinander kombinatorisch und funktionell abbildbar sind. Einerseits wird ein klingenartiger Knochenanker mit verdrehten Klingenflächen vorgesehen, welcher am Kopfbereich mindestens ein kugelähnliches Segment aufweist und andererseits dieser klingenartige Knochenanker eine innenliegende Gewindebuchse mit Knochengewinde besitzt. Die Gewindebuchse wird dabei nicht nur als Transportschraube verwendet, sondern dient hauptsächlich der Auszugsfestigkeit für die Osteosynthesevorrichtung. Die Gewindebuchse befindet sich dabei in einem eher proximal gelegenen Bereich der Klinge, so dass die Gewindebuchse mit ihrem Knochengewinde mit dem kortikalen Pedikelbereich im Eingriff steht. Die Gewindebuchse ist mithilfe eines Mitnehmerelements mit dem Schaft beziehungsweise dem Klingenbereich verbunden.

Die an der Osteosynthesevorrichtung wesentlich auftretenden Belastungen lassen sich in zwei Hauptkomponenten aufteilen; axiale Kräfte und Biegebeanspruchungen. Der Klingenbereich ist in der Lage die Biegebelastungen aufzunehmen und die Gewindebuchse entsprechend die Auszugskräfte. Dabei werden die Biegebelastungen hauptsächlich über den Schaft mit den Klingen abgefangen. Die Biegebelastung nimmt von distal nach proximal zu und wird vom Wirbelinneren auf den Schaft über die Kugel an den Implantats-TulpenKopf transferiert. Deshalb ist es erstrebenswert eine mechanisch optimierte Verbindung zwischen dem Kugelsegment und dem Klingenbereich des Knochenankers vorzusehen. Ein einstückiger Aufbau oder eine einstückige Verbindung zwischen Kugel und Klingenbereich ist deshalb ein biomechanisches Erfordernis. Der Stand der Technik offenbart hierzu keinen vergleichbaren Aufbau. Optional kann auch ein dazwischenliegender Halsbereich vorgesehen sein, um ein Verschwenken mit einem polyaxialen Tulpenkopf zu ermöglichen. Ein fehlender Halsbereich würde die Bewegungsfreiheit limitieren.

Zur Steigerung der Dauerfestigkeit am hochbelasteten Übergang vom Klingenbereich, über den Halsbereich zum Kugelsegment ist es vorteilhaft, wenn sich der Abstand zwischen den Flügelaußenkanten am proximalen Klingenbereich reduziert, so dass die Flügelaußenkanten zum Halsbereich in den Schaft münden. Dies hat neben einen stabilitätsfördernden Charakter auch die Funktion, dass die Osteosynthesevorrichtung überhaupt verschwenkbar ist, sobald sie mit einen polyaxialen Tulpenkopf montiert ist. Mit andersartig verlaufenden Flügelaußenkanten am proximalen Übergangsbereich würden die Flügelaußenkanten mit dem polyaxialen Tulpenkopf ein Impingement erzeugen und eine winkelige Auslenkung verhindern oder zumindest einschränken. Gleichzeitig ist es vorteilhaft, wenn am proximalen Klingenbereich sich der Schaft zum Halsbereich hin vergrößert.

Eine weitere Option der Steigerung der Dauerfestigkeit liegt in der Kompensation der fehlenden Struktur im Klingenbereich, nämlich dem Fenster für die Gewindebuchse. Dies kann dadurch erreicht werden, dass die Flügel ein oder mehr Verdickungen besitzen, die sich mindestens abschnittsweise parallel entlang der Schaft-Zentralachse erstrecken und mindestens den Bereich des Fensters überbrücken. Vorzugsweise variiert die Dicke der Verdickungen mindestens abschnittsweise entlang der Zentralachse. Ferner kann die Dicke der Verdickungen am proximalen Bereich größer sein, als die Dicke der Verdickungen am distalen Bereich. Die höchste Biegesteifigkeit der Klinge wird erzielt, wenn sich diese Verdickungen an den Flügelaußenkanten befinden. Dies hat den weiteren Vorteil, dass die Flügelaußenkanten mit ihren Verdickungen sich nicht in den kortikalen Knochen im Pedikelbereich einschneiden. Anatomisch betrachtet besitzt der Pedikel keine konzentrische Kontur, sondern näherungsweise ein Oval. Deshalb ist es vorteilhaft, wenn die Verdickungen an den Außenkanten mindestens abschnittsweise einem Oval entsprechen, um so eine optimale Anlagefläche von Klingenbereich und Pedikelbereich zu ermöglichen. Dies verhindert ein Einschneiden der Klinge in den kortikalen Knochen des Pedikels, was einen optimierten Lasttransfer gleichgestellt ist. Eine zusätzliche Möglichkeit zur Steigerung der Dauerfestigkeit der Osteosynthesevorrichtung ist eine Zunahme der Materialdicke der Flügelflächen von distal nach proximal.

Damit die Osteosynthesevorrichtung in der Lage ist Auszugskräfte aufnehmen zu können, muss die Gewindebuchse mindestens eine geeignete Fläche besitzen, die in Kontakt zu einer Fläche des Klingenbereichs steht, und gleichzeitig eine Kraft in axialer Richtung entlang der Zentralachse aufnehmen kann. Diese Kontaktflächen sind vorzugsweise innerhalb des Fensters angeordnet und stellen einen mechanischen Anschlag für die Gewindebuchse dar. Optimalerweise sind insgesamt zwei solcher mechanischen Anschläge, einer in distaler und einer in proximaler Richtung, an der Gewindebuchse vorgesehen. Vom polyaxialen Tulpenkopf ausgehend wird beispielsweise eine axiale Zugkraft auf die Osteosynthesevorrichtung und aufgrund der einstückigen Verbindung zwischen Kopf und Klinge auf den Klingenbereich abgegeben. Zwischen dem Klingenbereich und der Gewindebuchse existiert, wie zuvor erwähnt, mindestens ein mechanischer Anschlag. Aufgrund dieses Anschlags wird die axiale Zugkraft vom Knochenanker auf die Gewindebuchse übertragen. Die Gewindebuchse steht mit ihren Knochengewinde im direkten Eingriff mit dem kortikalen Pedikelknochen und gibt dadurch die initial eingeleitete Axialkraft auf den Knochen ab. Sind zwei mechanische Anschläge in dieser Konstruktion vorgesehen, wird auch eine bidirektionale Wirkung gegenüber axial eingeleiteter Zug- oder Druckkräfte erzielt.

Die Gewindebuchse ist rotatorisch innerhalb des Fensters im Klingenbereich gelagert. Ortsfest gehalten wird die Gewindebuchse mithilfe des Mitnehmerelements. Das Mitnehmerelement ist steckbar gestaltet, so dass durch Einstecken des Mitnehmerelements in den Schaft und durch die Gewindebuchse, die Gewindebuchse im Fenster des Klingenbereichs gehalten wird. Zusätzlich hat die steckbare Funktion den Vorteil, dass bei axialer Belastung der Gewindebuchse eine minimale Axialbewegung in Relation zum Mitnehmerelements erlaubt wird, da die bereits erwähnten Anschläge zwischen den Fensterflächen und der Gewindebuchse den Lasttransfer der axialen Kräfte sicherstellt. So wird das Mitnehmerelement in axialer Richtung nicht überansprucht bzw. ist sogar von der axialen Belastung her entkoppelt. Eine minimale Relativbewegung innerhalb der Anschläge kann aufgrund von Fertigungstoleranzen des Fensters und der Gewindebuchse notwendig sein.

Die Gewindebuchse ist in der Lage sich unabhängig vom Klingenbereich zu drehen. Dies wird dadurch erlaubt, dass das Mitnehmerelement mit der Gewindebuchse so in Kontakt steht, dass ein Drehmoment vom Mitnehmerelement auf die Gewindebuchse übertragen werden kann. Hierfür kommen Profile, die miteinander Kontakt stehen zum Einsatz, die dazu geeignet sind ein Drehmoment zu übertragen und gleichzeitig ineinander steckbar sind. Dazu gehören alle aus dem Stand der Techniken bekannten lastübertragenden Profile, wie beispielsweise flächige Profile (z.B. Einflach, Zweiflach, ..., Sechskant, ..., Polykant) oder auch Zahnprofile wie beispielsweise Torx. Als Alternative stehen andere Fügeverfahren zur Auswahl, wie beispielsweise Kleben, Verstiften, Schweißen, Verpressen usw. Zur Einleitung eines Drehmoments über das Mitnehmerelement ist es vorteilhaft, wenn das Mitnehmerelement eine Werkzeugansatzstelle besitzt, wo auch hier alle aus dem Stand der Technik bekannten Ein- oder Aufsätze denkbar sind. Für einen selbstschneidenden Charakter der Gewindebuchse ist es von Vorteil, wenn das außenliegende Knochengewinde sogenannte Schneidkanten besitzt. Optimalerweise sind sie so gestaltet, dass beim Einschlagen des Klingenbereichs in den Knochen, das Gewinde beginnt im Knochen zu greifen. Zur Reduktion der Anzahl der Eindrehwindungen der Gewindebuchse ist es von Vorteil, wenn das Gewinde doppel- oder mehrgängig gestaltet ist. Eine eingängige Version ist dabei nicht ausgeschlossen.

Wie bereits erwähnt, ist das Mitnehmerelement steckbar im Schaft und in der Gewindebuchse vorgesehen. Damit das Mitnehmerelement verliersicher im Schaft gehalten wird, ist optionaler Weise eine Verrastung zwischen Mitnehmerelement und Schaft vorgesehen. Durch ein Zusammenstecken der Komponenten ist der Zusammenbau der Osteosynthesevorrichtung relativ einfach. Natürlich sind auch andere Verbindungs- und Sicherungsmechanismen denkbar.

Sollte der Kopf des Mitnehmerelements über den sphärischen Durchmesser des Kugelsegments hinausstehen, kann durch das winkelstabile Verklemmen des polyaxialen Tulpenkopfes sogar eine Blockade der Rotation des Mitnehmerelements und der Gewindebuchse erreicht werden. Wird der Tulpenkopf wieder gelockert, löst sich diese Blockade wieder.

Zur weiteren Steigerung der Biegefestigkeit des Klingenbereichs ist es vorteilhaft, wenn das Mitnehmerelement einerseits im proximalen Bereich im Schaft gelagert ist und sich durch die Gewindebuchse erstreckt und anderseits nach distal aus der Gewindebuchse herausragt und im Klingenbereich distal vom Fenster ebenfalls gelagert ist. Dadurch befindet sich im Schaft ein zusätzliches Element, welches eine Biegebelastung aufnehmen kann. Damit wird die Fehlstelle des Fensters von innen überbrückt und die Biegesteifigkeit erhöht. Es kann auch hilfreich sein, wenn das Fenster einen optionalen Steg besitzt, der eine zusätzliche Verstrebung im Fenster und damit eine zusätzliche Biegesteifigkeit des Schafts bereitstellt.

Spezielle Größenbezüge sind wichtig einzuhalten, da ein tatsächlich funktionierender Aufbau aus innenliegender Gewindebuchse, klingenartigen Knochenanker und steckbaren Mitnehmerelement erreicht werden soll. Es muss ein Kopf mit Kugelsegment vorgesehen sein, welcher einstückig mit dem Klingenbereich eine mechanische Einheit bildet; dem Knochenanker. Des Weiteren befindet sich für die Gewindebuchse im Klingenbereich ein Fenster. Dies sorgt bereits für eine geometrische Situation, die nur dann aufzulösen ist, wenn unterschiedliche Merkmale und Bezüge zu einander gemäß den erfindungsgemäßen Ansprüchen eingehalten werden.

Betrachtet man die Anatomie der Wirbelsäule von der frontalen Ebene aus, ist zu erkennen, dass die posterioren Strukturen, nämlich die Pedikel, im Schnitt ein Oval approximieren und das Oval eine Schwerpunktachse besitzt. Im posterior-anterioren Schnittverlauf der Pedikelstrukturen ist zu erkennen, dass diese Oval-Schwerpunktachse ihren Orientierungswinkel ändert, und zwar links und rechts unterschiedlich d.h. gegenläufig. Dadurch ist es notwendig zu wissen, welche Verschränkungsrichtung die Flügelverdrehung des Klingenbereichs aufweist, damit die Osteosynthesevorrichtung anatomisch richtig in den richtigen Pedikel eingebracht werden kann. Das heißt, es ist wichtig bei der Osteosynthesevorrichtung zwischen einer linken und einer rechten Version strikt zu unterscheiden, da sich diese in der Richtung der Flügelverschränkung unterscheiden. Erst dieser Unterschied und eine entsprechende Markierung oder Beschriftung, wie beispielsweise "R" für Rechts oder "L" für Links, holt das biomechanisch Bestmögliche aus dem Erfindungspotential. Eine falsche Kombination kann schlimmstenfalls während der Implantation zum Durchbruch der Pedikelwand führen.

Naturgemäß besitzen die Pedikelkanäle einen gewissen Formfaktor, welcher das Oval beschreibt. Dieser Formfaktor definiert den Zusammenhang aus Höhe und Breite. Optimalerweise sind die erfindungsgemäßen Osteosynthesevorrichtungen genau daran angepasst, damit sie den ovalen Querschnitt am besten wiedergeben. Dabei weist, die Osteosynthesevorrichtung, mit den beiden Flügeln (15, 16) eine Breite (1516), die zwischen den Außenkanten der Flügel (154, 164) definiert ist, und einen Außendurchmesser (252) des Knochengewindes, einen Formfaktor mit dem Verhältnis aus 1516/252 auf, welcher zwischen 1,3 bis 2,5, vorzugsweise 1,4 bis 2,2, vorzugsweise 1,6 bis 2,0 beträgt.

Als zusätzliches Mittel zur Erhöhung der Verankerungsstabilität im Knochen, falls die Knochenqualität zu niedrig ist, ist eine Kanülierung mit seitlichen Öffnungen vorgesehen. Durch diese Öffnungen kann Knochenzement injiziert werden. Vorteilhaft ist hier, dass die Orientierung der seitlichen Öffnungen nach der Implantation immer nach kranial und kaudal zeigen, wohin die größte Last innerhalb der Spongiosa gerichtet ist. Des Weiteren erweist es sich als vorteilhaft, wenn die zentrale Kanülierung unterschiedliche Durchmesser besitzt, um einen Zementaustritt nach distal entgegenzuwirken.

Der Tulpenkopf besteht aus einem in der Seitenansicht u-förmigen Gabelkopf, der in proximaler Richtung zwei Gabelschenkel mit einem innenliegenden Gewinde aufweist, und darin der Verbindungsstab aufgenommen werden kann, und in dem innenliegenden Gewinde eine Madenschraube geführt ist, und der Gabelkopf lösbar mit der Osteosynthesevorrichtung verbunden ist. Des Weiteren ist die Osteosynthesevorrichtung schwenkbar im Kugelsitz des Gabelkopfs gelagert, wobei der Gabelkopf am Kugelsitzbereich so gestaltet ist, dass die Osteosynthesevorrichtung mit ihrem Kugelsegment von distal kommend montiert wird.

Implantiert werden soll die Osteosynthesevorrichtung indem die Flügelausrichtung des distalen Endes des Knochenankers der Hauptausrichtung des Pedikelkanals entspricht. Dies entspricht nahezu einer kranial-kaudalen Ausrichtung. Die Osteosynthesevorrichtung wird durch kurze Schläge in den Pedikelkanal bis zur Höhe der Gewindebuchse eingetrieben. Sobald die Gewindebuchse Kontakt mit dem Knochen hat, wird über die Werkzeugansatzstelle des Mitnehmerelements ein Drehmoment eingeleitet, was zur Rotation und damit zum Vortrieb der Gewindebuchse im Knochen führt. Dadurch wird die Osteosynthesevorrichtung in den Wirbelkörper transportiert. Während des gesamten Vorgangs der Implantation dreht sich das Knochenverankerungselement um die Zentralachse gemäß der definierten Verschränkung der Flügelflächen im Klingenbereich. In der finalen Position besitzt die distale Flügelausrichtung eine lateral-mediale Ausrichtung, wobei die proximale Flügelausrichtung die der Pedikelhauptausrichtung entspricht.

### Kurze Beschreibung der Zeichnungen zeigen

Fig. 1 die erfindungsgemäße Osteosynthesevorrichtung in einer Schrägansicht.
Fig. 2 der alleinige Knochenanker der erfindungsgemäßen Osteosynthesevorrichtung in einer Seitenansicht und mit diversen Schnitten. Aus Gründen einer besseren Darstellung wurde hier auf die Verdrehung der Flügel verzichtet.
Fig. 3 die erfindungsgemäße Osteosynthesevorrichtung aus Fig. 1 in Explosionsdarstellung.
Fig. 4 die Gewindebuchse mit eingestecktem Mitnehmerelement ohne den Knochenanker.
Fig. 5 die vollständige erfindungsgemäße Osteosynthesevorrichtung in Seitenansicht und im Schnitt. Auch hier wurde aus Darstellungsgründen auf die Verdrehung der Flügel verzichtet.
Fig. 6 eine alternative Ausgestaltungsform, bei der die Verrastung am Kopfbereich vorgesehen ist.
Fig. 7 die erfindungsgemäße Osteosynthesevorrichtung in einer anderen Schrägansicht
Fig. 8. Eine alternative Ausführungsform mit zusätzlichem Steg und zwei Gewindebuchsen im Fenster zur Erhöhung der Steifigkeit.
Fig. 9 zeig zwei erfindungsgemäße Osteosynthesevorrichtungen, die eine unterschiedliche Ausrichtung der Flügelverdrehung besitzen.
Fig. 10 zeigt die Montage aus unterschiedlichen Osteosynthesevorrichtungen zu einem Konstrukt, wie es bei Wirbelsäuleneingriffen zum Einsatz kommt.

### Beschreibung

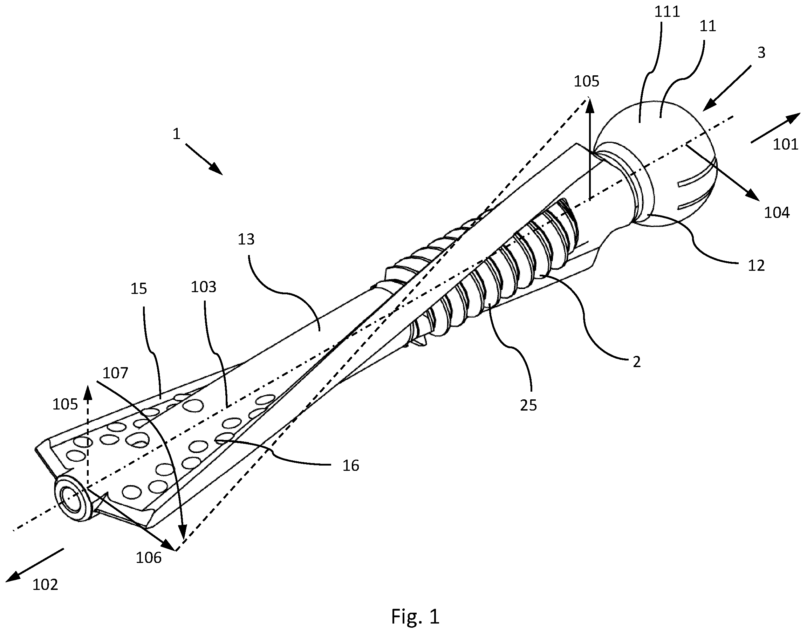

Für die Osteosynthesevorrichtung (10), sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert sich die radiale Ausbreitung (104). Die umfängliche Ausbreitung ist durch einen konstanten Radius und entlang eines variablen Umfangswinkels definiert (Fig. 1). Die Osteosynthesevorrichtung (10) ist hauptsächlich für die Fixation von Knochenkomponenten und Knochenfragmenten, insbesondere Wirbel vorgesehen. Sie besteht aus einem Knochenanker (1), und dieser besteht aus einem Schaft (13), welcher sich entlang der Zentralachse (103) erstreckt und dadurch eine distale (102) und eine proximale (101) Richtung definiert. Der Knochenanker (1) besitzt wenigstens einen Klingenbereich (14) mit einen ersten (15) und einen zweiten Flügel (16), und grenzt proximal (101) an einen Halsbereich (12) und im weiteren Verlauf einen Kopf (11) mit mindestens einem Kugelsegment (111) an. Ein wesentliches erfindungsgemäßes Merkmal ist, dass der Schaft (13) mit dem Klingenbereich (14), dem Halsbereich (12) und dem Kopf (11) mit Kugelsegment (111) insgesamt einstückig ausgebildet ist. Die Osteosynthesevorrichtung (10) ist außerdem dadurch charakterisiert, dass der Klingenbereich (14), mit den beiden Flügeln (15, 16) am proximalen Bereich (101) eine erste Flügelausrichtung (105) besitzt und die Flügel (15, 16) am distalen Ende (102) eine zweite Flügelausrichtung (106) aufweisen, die unterschiedlich zur ersten Flügelausrichtung (105) ist, wobei dieser Unterschied über einen Verschränkungswinkel (107) beschreibbar ist.

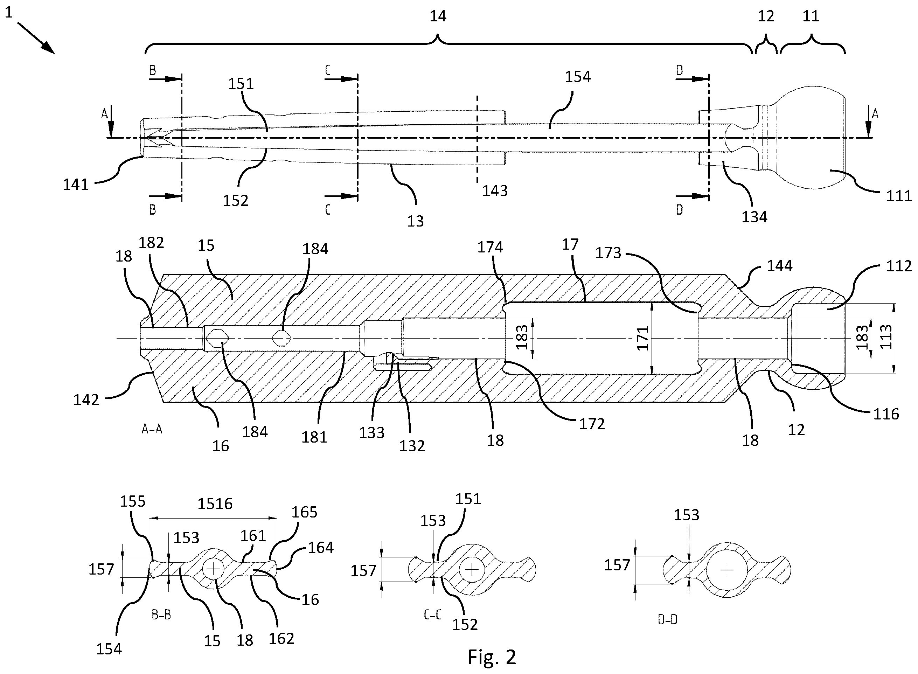

In Fig. 2 ist dargestellt, dass der Knochenanker (1) der Osteosynthesevorrichtung (10) eine zentrale und durchgängige Kanülierungsöffnung (18) mit einem Durchmesser (183) aufweist, und im Klingenbereich (14) mindestens ein Fenster (17) mit einer Öffnungsbreite (171) vorgesehen ist, und das Fenster (17) mit der Kanülierungsöffnung (18) kommuniziert, und der Kopfbereich (11) eine Öffnung (112) mit einem Öffnungsdurchmesser besitzt (113), welche ebenfalls mit der Kanülierungsöffnung (18) kommuniziert. Dabei ist der Durchmesser der Kanülierungsöffnung (183) kleiner ist als die Öffnungsbreite des Fensters (171) und der Durchmesser der Kanülierungsöffnung (183) ebenfalls kleiner ist als der Öffnungsdurchmesser am Kopf (113). Außerdem ist es vorteilhaft, wenn die Öffnungsbreite des Fensters (171) mindestens gleich groß oder größer als der Öffnungsdurchmesser am Kopf (113) ist. Dies gilt insbesondere dann, wenn Gewindebuchsen mit größerem Durchmesser notwendig sind. Dabei verläuft zwischen der Öffnung am Kopf (112) und der Kanülierungsöffnung (18) wenigstens ein Wandungsabschnitt oder Reihe von Wandungsabschnitten (116), welche beide Öffnungen miteinander verbindet und diese Wandungsabschnitte (116) innerhalb des Kopfbereichs (11) liegen.

Die zentrale Kanülierung (18) kann zur Erfüllung zweier Aufgaben eingesetzt werden. Einerseits kann die erfindungsgemäße Osteosynthesevorrichtung (10) minimal-invasiv über einen Führungsdraht geführt in den Knochen implantiert werden und anderseits kann Knochenzement durch die Kanülierung (18) von proximal injiziert werden.

Zum Verhindern einer Zementleckage nach distal ist es vorteilhaft, wenn die Kanülierung (18) in distaler Richtung (102) wenigstens eine Verjüngung (181, 182) aufweist. Der Zement kann dabei über Fenestrationsöffnungen (184) im Klingenbereich (14) in das Knochengewebe austreten. Diese Fenestrationsöffnungen (184) kommunizieren mit der Kanülierung (18) und sind entlang einer Sekante oder Flächennormalen der Flügelaußenflächen (151, 152, 161, 162) ausgebildet.

Das im Klingenbereich (14) vorgesehene Fenster (17) sorgt für eine strukturelle Schwächung der Osteosynthesevorrichtung (10), vor allem in Biegerichtung. Zur Kompensation der Biegesteifigkeit des Schafts mit Klingenbereich (14) ist es vorteilhaft, dass die Flügel (15, 16) innerhalb des Klingenbereichs (14) abschnittsweise mindestens eine Verdickung (155, 165) besitzen, die hauptsächlich parallel zur Zentralachse (103) verläuft, und zur Erhöhung der Biegesteifigkeit der Osteosynthesevorrichtung (10) beiträgt. Die Verdickung kann als longitudinales Profil entlang einer Parallelen zur Zentralachse (103) innerhalb der Flügelflächen ausgebildet sein. Denkbar sind beispielsweise längliche Streben die sich entlang der Flügel erstrecken und einen beliebigen Abstand zur Zentralachse aufweisen. In Fig. 2 ist eine bevorzugte Ausführungsform der erfindungsgemäßen Osteosynthesevorrichtung (10) dargestellt, bei der diese Verdickungen (155, 165) an der Außenkante (154, 164) der Flügel (15, 16) lokalisiert sind, und eine größere Dicke (157) als der Flächenabstand der Flügelflächen (153) aufweisen. Dadurch wird der größte Stabilitätsgewinn erzielt. Des Weiteren ist es vorteilhaft, dass die Außenkanten (154, 164) mindestens eine konvexe Krümmung besitzen und diese Krümmungen mindestens abschnittsweise ein Oval approximieren. Mit dem approximierten Oval erzeugen die Außenkanten (154, 164) eine homogenisierte Kontaktzone mit reduzierten Kontaktspannungen zum kortikalen Pedikelknochen in kranial-kaudaler Richtung. Dadurch kann sich die erfindungsgemäße Osteosynthesevorrichtung an der kortikalen Pedikelwandung abstützen und so Kräfte besser aufnehmen.

Da bei einer Biegebeanspruchung die proximalen Strukturen der Osteosynthesevorrichtung (10) am höchsten belastet werden, ist es aus mechanischer Sicht vorteilhaft, dass die Dicke (157) der Verdickungen (155, 165) am proximalen Bereich (101) größer ist als die Dicke (157) der Verdickungen (155, 165) am distalen Bereich (102). Das gleiche gilt auch für den Flächenabstand der Flügelflächen (153) innerhalb des Klingenbereichs. Auch dieser kann mindestens abschnittsweise entlang der Zentralachse (103) variieren, um so gezielt der Biegesteifigkeit beizutragen.

Für die Erhöhung der Biegesteifigkeit der Osteosynthesevorrichtung (10) ist es ebenfalls von Vorteil, dass innerhalb des Klingenbereichs (14) der Kerndurchmesser (131) des Schafts (13) im proximalen Klingenbereich (134) zunimmt. Außerdem positiv ist, dass die Klingenbreite (1516) im proximalen Klingenbereich (144) abnimmt und im Halsbereich (12) im Kerndurchmesser des Schafts mündet, um Spannungsspitzen am Übergang vom Klingenbereich (14) zum Halsbereich (12) zu verhindern. Zur Reduktion mit Spannungskonzentrationen im Fensterbereich können optional Rundungen oder anderweitig vorgesehene Übergänge (174) am Fensterausschnitt vorgesehen werden.

Zum besseren Einbringen in den Wirbel ist es vorteilhaft, wenn die distale Spitze des Klingenbereichs an jeder Flügelfläche eine Schneidkante (142) aufweist, die entweder einen symmetrischen oder asymmetrischen Anschnitt besitzt. Optimalerweise besitzt die Schneidkante (142) einen spitzen Schneidwinkel. Orthogonal hierzu betrachtet, ist es vorteilhaft, wenn die Schneidkanten beider Flügelflächen im stumpfen Winkel zu einander stehen. Des Weiteren ist es erstrebenswert, wenn die distale Spitze (141) des Schafts (13) in distaler Richtung hervorstehend ist. Dadurch kann die Osteosynthesevorrichtung (10) mit der distalen Schaftspitze (141) als erstes Kontaktelement in einen Bohrkanal im Knochen geführt werden. Ohne dieses Merkmal ist eine solche Führung erschwert.

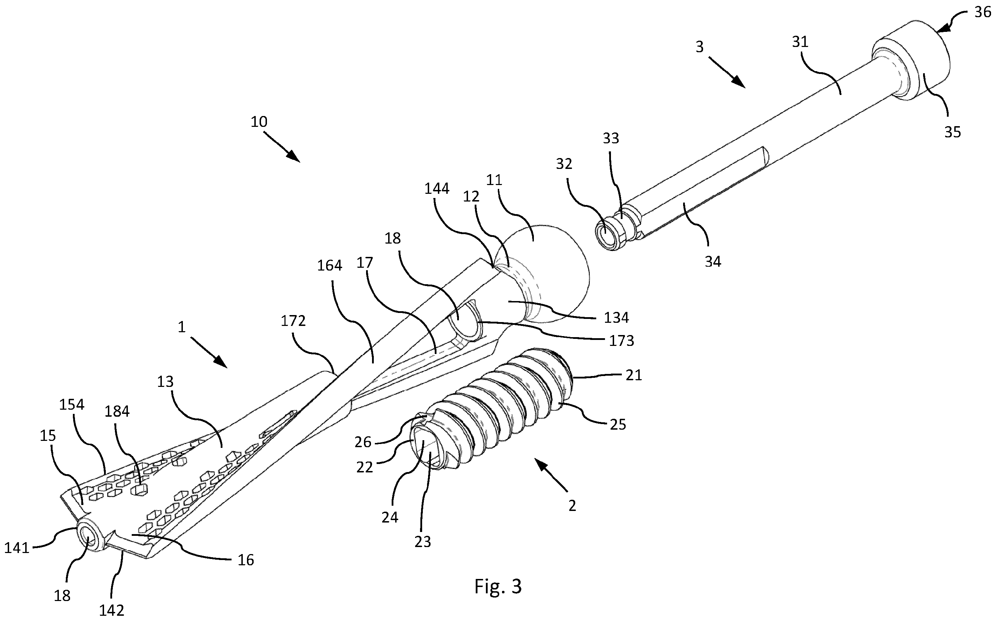

Fig. 3 zeigt die erfindungsgemäße Osteosynthesevorrichtung (10) mit dem Fenster (17) und dass das Fenster (17) innerhalb des Klingenbereichs positioniert ist und sich näher zum proximalen Ende (144) des Klingenbereichs als zum distalen Ende (141) des Klingenbereichs (102) befindet. Im Fenster (17) ist eine Gewindebuchse (2) mit einem Knochengewinde (25) rotatorisch gelagert. Das Knochengewinde (25) kann wenigstens eine Schneidkante (26) aufweisen, damit das Gewinde einen selbstschneidenden Charakter erhält. Die Position des Fensters (17) ist dabei so gewählt, dass die Gewindebuchse (2) hauptsächlich mit dem kortikalen Knochen im Pedikelbereich verzahnt ist, sobald die Osteosynthesevorrichtung (10) vollständig implantiert ist. Die Gewindebuchse (2) besitzt eine zentrale Öffnung (23), wobei der Durchmesser der zentralen Öffnung (23) in etwa gleichgroß zum Kanülierungsdurchmesser (183) des Schafts (13) ist. Dadurch ist es möglich, dass ein Mitnehmerelement (3) steckbar mit dem Schaft (13) und der Gewindebuchse (2) vorgesehen ist. Das Mitnehmerelement (3) besitzt einen Kopf (35) und darin eine Werkzeugansatzstelle (36) und einen länglichen Schaft (31). Der längliche Schaft (31) ist nachdem Zusammenstecken in der Kanülierung (18) des Schafts (13) und in der Gewindebuchse (2, 23) gelagert. Des Weiteren ist zu erkennen, dass das Mitnehmerelement (3) am Schaftbereich (31) abschnittsweise mindestens ein Profil (34) aufweist und die Gewindebuchse (2) abschnittsweise wenigstens ein dazu kongruentes Profil (24) aufweist, welche miteinander im Eingriff stehen und dazu geeignet sind, ein Drehmoment von der Werkzeugansatzstelle (36) auf die Gewindebuchse zu übertragen.

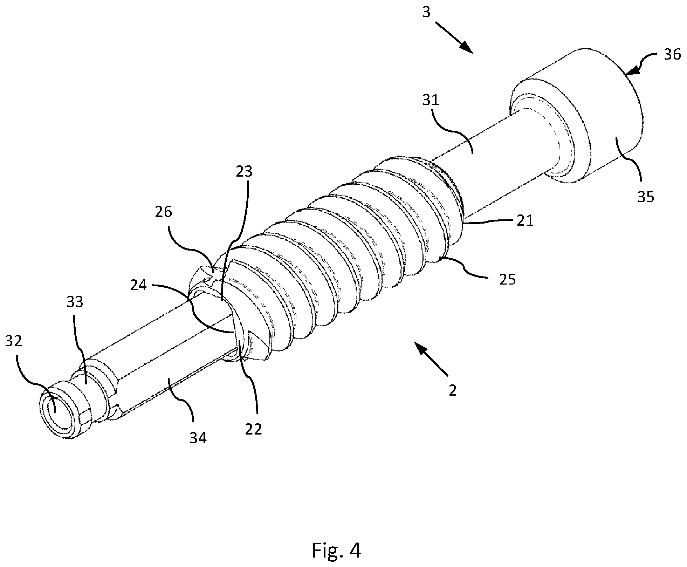

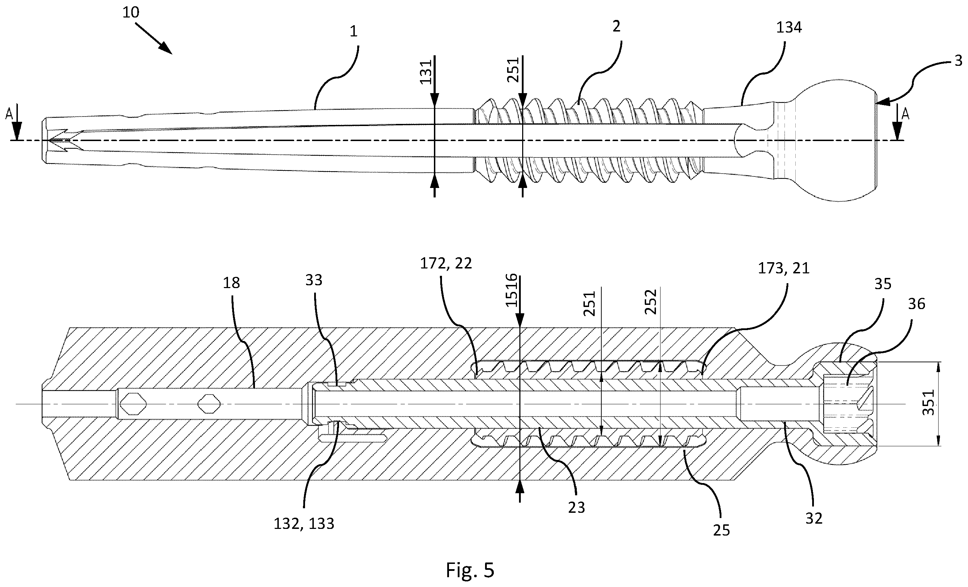

Fig. 4 zeigt ohne die Darstellung des Knochenankers (1), wie die Gewindebuchse (2) und das Mitnehmerelement (3) zusammengesteckt sind. Ein wesentliches Merkmal ist, dass der Schaft (31) des Mitnehmerelements nach distal (102) aus der Gewindebuchse (2) herausragt und dieses herausragende distale Schaftende in der Kanülierung (18) des Schafts (13) des Knochenankers (1) gelagert ist, insbesondere im Klingenbereich (14). Damit besitzt der Schaft (31) mindestens zwei Lagerpositionen innerhalb der Kanülierung (18) des Schafts (13), distal und proximal von der Gewindebuchse (2). Dadurch kann das Mitnehmerelement (3) eine Biegebeanspruchung des Schafts (13) zusätzlich aufnehmen. Hier ebenfalls dargestellt ist, dass das Mitnehmerelement (3) eine Kanülierungsöffnung (32) besitzt und diese im montierten Zustand mit der Kanülierungsöffnung (18) des Schafts (13) kommuniziert (Fig. 5). Erst dadurch wird erreicht, dass die gesamte Osteosynthesevorrichtung (10) kanüliert ist.

Fig. 5 zeigt, dass die Gewindebuchse (2) eine distale Fläche (22) besitzt und diese distale Fläche (22) in direktem Kontakt mit einer Anlagefläche (172) der Fensterung (17) steht und dieser Kontakt als Anschlag dient. Des Weiteren wird gezeigt, dass die Gewindebuchse (2) eine proximale Fläche (21) besitzt und diese proximale Fläche (21) in direktem Kontakt mit einer Anlagefläche (173) der Fensterung (17) steht und dieser Kontakt als Anschlag dient. Mit Hilfe dieser Anschläge ist es möglich, dass die von der Gewindebuchse (2) aufgenommenen Auszugskräfte auf den Klingenbereich (14) und dann auf den Knochenanker (1) und schließlich auf das Kugelsegment (111) übertragen werden können. Vorzugsweise sind diese Anschläge als konzentrisch planare Kontaktflächen gestaltet, die gleichzeitig eine freie Rotation der Gewindebuchse (2) erlauben. Wichtig für den Aufbau der erfindungsgemäßen Osteosynthesevorrichtung (10) sind verschiedene geometrische Bezüge, nämlich, dass die Gewindebuchse (2) einen Gewindekerndurchmesser (251) aufweist, welcher in etwa dem Außendurchmesser des Schafts (131) entspricht. Weiterhin, muss sichergestellt sein, dass das Mitnehmerelement (3) einen Kopf (35) bereitstellt und dieser Kopf einen Außendurchmesser (351) besitzt, welcher in etwa gleich groß oder kleiner als der Gewindeaußendurchmesser (252) der Gewindebuchse (2) ist. Erst dadurch ist es möglich ein Portfolio an Osteosynthesevorrichtungen (10) mit verschiedenen Gewindebuchsen-Außendurchmesser (252) bereitzustellen, die auch für die patientenspezifisch unterschiedlichen Pedikelgrößen passen. Bei der Größenvarianz der Osteosynthesevorrichtungen (10) ist es vorteilhaft, wenn das Mitnehmerelement (3) als auch die Größe das Halsbereichs (12) und des Kopfbereichs (11) stets gleichgroß bleiben. Somit können für die unterschiedlichen Osteosynthesevorrichtungen (10) dieselben Tulpenköpfe (4) zur Adaption verwendet werden. Mit der Varianz des Gewindebuchsen-Außendurchmessers (252) muss die Größe des Klingenbereichs ebenfalls angepasst werden, damit die Relation aus Gewindebuchsen-Außendurchmesser (252) und Klingenbreite (1516) anatomisch stimmig ist. Dabei muss beachtet werden, dass der Klingenbereich (14), mit den beiden Flügeln (15, 16) eine Breite (1516) zwischen den Außenkanten (154, 164) der Flügel definiert, und ein Gewindeaußendurchmesser (252) der Gewindebuchse (2) definiert ist, wobei der Formfaktor, aus dem Verhältnis aus 1516/252, zwischen 1,3 bis 2,5, vorzugsweise 1,4 bis 2,2, vorzugsweise 1,6 bis 2,0 beträgt.

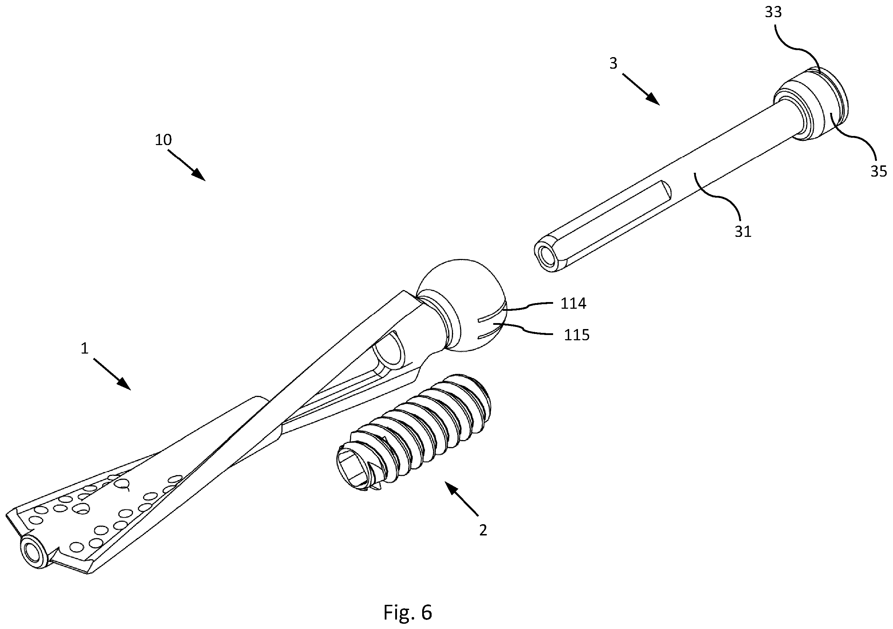

In Fig. 5, ist wie zuvor erwähnt, zu erkennen, dass das Mitnehmerelement (3) eine Kanülierung (32) besitzt und diese Kanülierung (32) mit der Kanülierungsöffnung (18) des Schafts (13) kommuniziert. Des Weiteren wird hier ein Rastmechanismus gezeigt, bei welchem mindestens ein elastisches Halteelement (132) innerhalb des Klingenbereichs (14) (Fig. 5) oder außerhalb des Klingenbereichs (115, 11) (Fig. 6) vorgesehen ist, welches mit dem Mitnehmerelement (3, 33) verrastet wird, was einerseits die werkzeugfreie Montage des Mitnehmerelements (3) ermöglicht und anderseits eine Verliersicherung des Mitnehmerelements (3) in axialer Richtung bereitstellt. Alternativ, aber nicht dargestellt, kann auch mindestens ein elastisches Halteelement am Mitnehmerelement (3) vorgesehen sein, welches mit dem Schaft (13) auf eine beliebige Weise verrastet wird. Auf Rastmechanismen, die aus dem Stand der Technik bekannt sind, soll hier nicht eingegangen werden.

Fig. 6 zeigt eine alternative Ausführungsform des Rastmechanismus, wobei das elastisches Halteelement (115) als Federelement gestaltet ist, welches durch zwei Schlitze (114) erzeugt wird. Auf der Innenseite des elastischen Halteelements (115) ist ein Hakenprofil ausgebildet, welches mit einer Rastnut (33) am Kopf (35) des Mitnehmerelements (3) verrastet werden kann. Eine umgekehrte Variante ist ebenfalls denkbar, bei der das elastische Halteelement am Kopf (35) des Mitnehmerelements (3) und eine Nut im Kopf (11) vorgesehen ist.

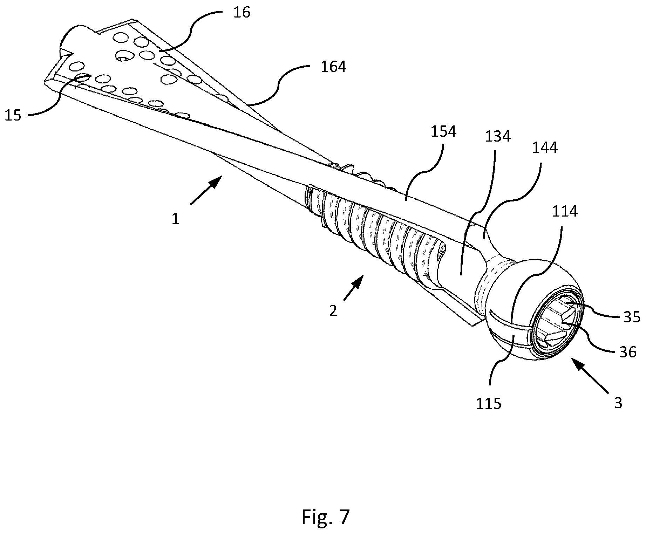

In Fig. 7 ist eine final montierte Osteosynthesevorrichtung (10) dargestellt. Zu erkennen ist die Werkzeugansatzstelle (36), die im Kopf (35) des Mitnehmerelements (3) vorgesehen ist. Eine Drehung der Werkzeugansatzstelle mit einem dazu geeigneten Instrument, erzwingt eine Rotation der Gewindebuchse (2). Ansonsten ist der Knochenanker (1) starr und einstückig ausgeformt, um so ein Maximum an Stabilität zu bieten.

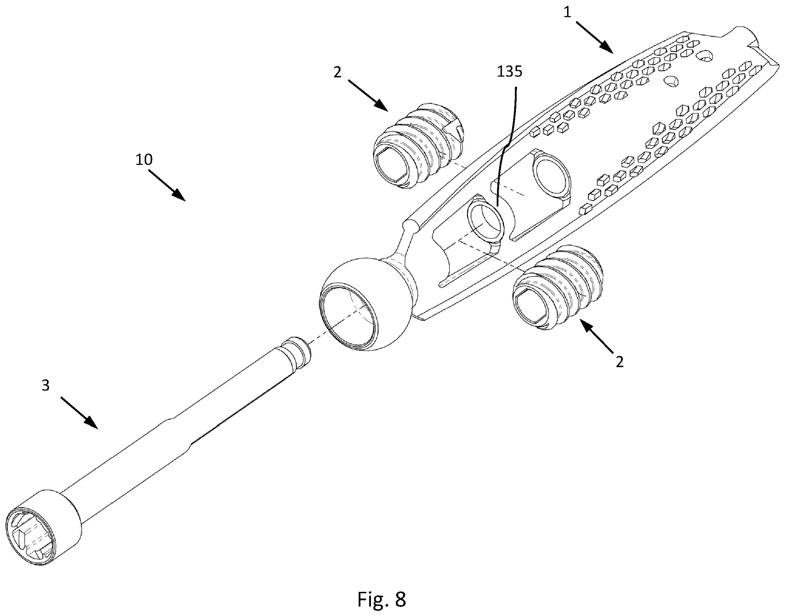

Fig. 8 stellt eine alternative Ausführungsform vor, bei der zwei Gewindebuchsen (2) vorgesehen sind, die durch einen Steg (135) getrennt sind. Die Breite des Stegs (135) muss dabei im faktoriellen Bereich der Gewindesteigung der Gewindebuchsen (2) liegen, damit die proximale Gewindebuchse im selben Gewindebett beim Einschrauben läuft, die die distale Gewindebuchse beim Einschrauben zuvor kreiert hat. Der Steg (135) hat die Aufgabe, ein zusätzliches Lager für das Mitnehmerelement (3) bereitzustellen und dadurch die Biegesteifigkeit des Knochenankers (1) zu erhöhen.

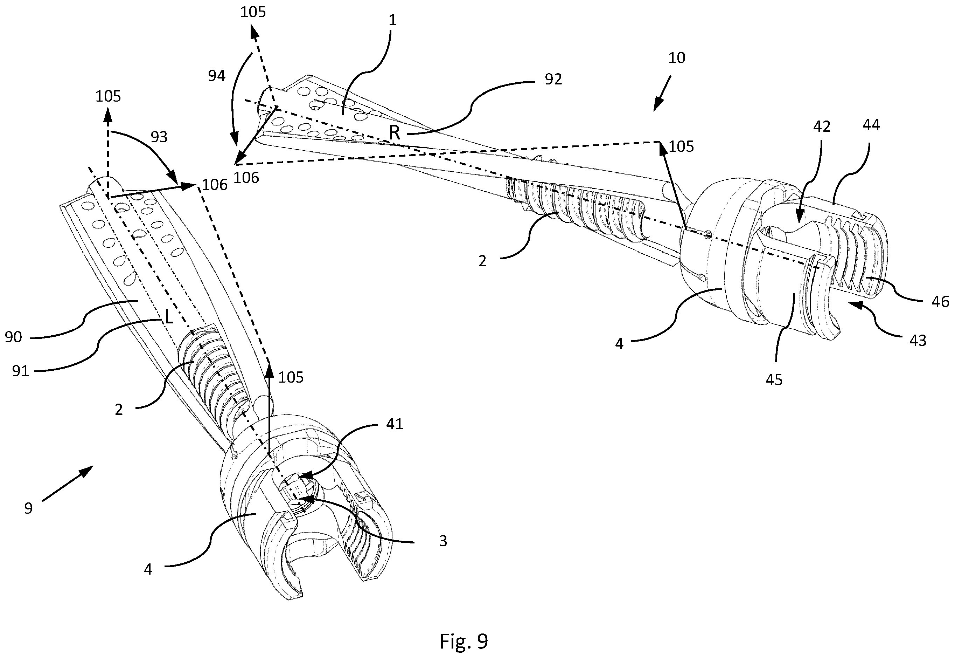

Fig. 9 zeigt, wie eingangs erwähnt, dass die Osteosynthesevorrichtung (10) in Kombination mit einem polyaxial verschwenkbaren Tulpenkopf (4) eingesetzt werden kann. Der polyaxial verschwenkbare Tulpenkopf (4) besitzt einen in der Seitenansicht durch zwei Schenkel (44, 45) geformten u-förmigen Ausschnitt (42), welcher für die Aufnahme eines Verbindungsstabs (6) geeignet ist und ein innenliegendes proximales Gewinde (46) für ein Fixierungselement (5) besitzt.

Ebenfalls eingangs erwähnt wurde, dass die Pedikelkanäle im Schnitt einen unterschiedlichen Verlauf des linken und rechten Pedikelprofils aufweisen. Deshalb ist es vorteilhaft, wenn bei einem Portfolio aus Osteosynthesevorrichtungen (9, 10) jeweils eine links und eine rechte Version eines Knochenankers (1, 90) vorgesehen wird. Genauer beschrieben heißt das, dass der Klingenbereich (14), mit den beiden Flügeln (15, 16) am proximalen Bereich (101) eine erste Flügelausrichtung (105) besitzt und die Flügel (15, 16) am distalen Ende (102) eine zweite Flügelausrichtung (106) aufweisen, die unterschiedlich zur ersten Flügelausrichtung (105) ist, wobei dieser Unterschied über einen Verschränkungswinkel (107) beschreibbar ist, und aus proximal kommender Blickrichtung betrachtet, die Verschränkung entgegen des Uhrzeigersinns (94) gerichtet ist. Zusätzlich gilt, dass der Klingenbereich (14), mit den beiden Flügeln (15, 16) am proximalen Bereich (101) eine erste Flügelausrichtung (105) besitzt und die Flügel (15, 16) am distalen Ende (102) eine zweite Flügelausrichtung (106) aufweisen, die unterschiedlich zur ersten Flügelausrichtung (105) ist, wobei dieser Unterschied über einen Verschränkungswinkel (107) beschreibbar ist, und aus proximal kommender Blickrichtung betrachtet die Verschränkung im Uhrzeigersinn (93) gerichtet ist. Vorteilhaft ist, wenn die Osteosynthesevorrichtung eine Markierung oder Beschriftung besitzt, die einen Hinweis auf die Richtung der Verschränkung oder einen Hinweis zur anatomischen Platzierung (91, 92) bereitstellt. In der Fig. 9 ist exemplarisch dargestellt, wie die unterschiedlichen Versionen mit "R" und "L" für Rechts und Links (91, 92) gekennzeichnet sind. Alternative Markierungen oder Farbgebungen sind ebenfalls denkbar.

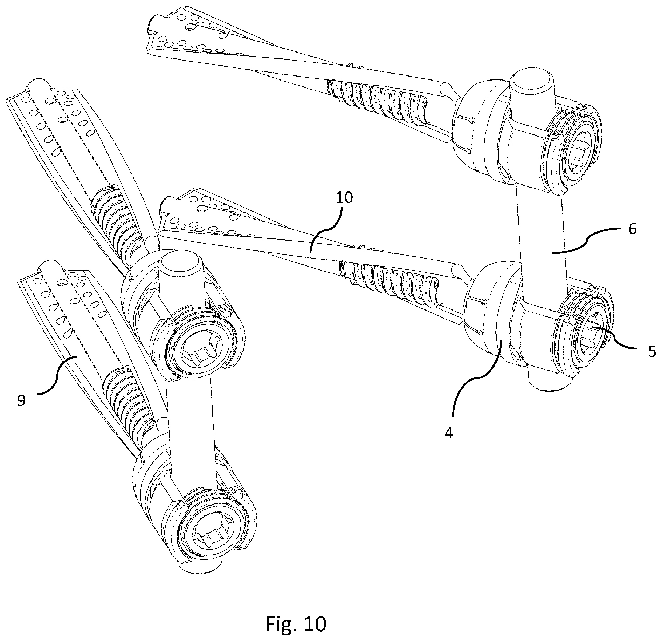

Bei der Zusammenstellung eines Systems aus mindestens zwei Osteosynthesevorrichtungen (9, 10) muss beachtet werden, dass mindestens eine der Osteosynthesevorrichtungen (9) eine Verschränkung der Flügelausrichtungen im Uhrzeigersinn (93) und mindestens eine weitere Osteosynthesevorrichtung (10) eine Verschränkung der Flügelausrichtungen entgegen dem Uhrzeigersinn (94) aufweist (Fig. 9 und Fig. 10).

Die Osteosynthesevorrichtung kann in Kombination mit einem polyaxialen Tulpenkopf verwendet werden, damit zwei oder mehr Osteosynthesevorrichtungen mit Tulpenkopf zu einem rigiden Konstrukt mit Hilfe von Verbindungsstäben miteinander montiert werden, damit die knöchernen Strukturen stabilisiert werden (Fig. 10).

## Patentansprüche

1. Osteosynthesevorrichtung (10) für die Fixation von Knochenkomponenten und Knochenfragmenten, insbesondere Wirbel, bestehend aus einem Knochenanker (1), wobei der Knochenanker (1) einen Schaft (13) besitzt, welcher sich entlang einer Zentralachse (103) erstreckt und dadurch eine distale (102) und eine proximale (101) Richtung definiert, und der Schaft (13) wenigstens einen Klingenbereich (14) mit einen ersten (15) und einen zweiten Flügel (16) besitzt, und proximal (101) an einen Halsbereich (12) angrenzt und im weiteren Verlauf einen Kopf (11) mit mindestens einem Kugelsegment (111) besitzt, und der Knochenanker (1) eine zentrale und durchgängige Kanülierungsöffnung (18) mit einem Durchmesser (183) aufweist, und im Klingenbereich (14) mindestens ein Fenster (17) mit einer Öffnungsbreite (171) vorgesehen ist, wobei im Fenster (17) eine Gewindebuchse (2) mit einem Knochengewinde (25) rotatorisch beweglich gelagert ist und die Gewindebuchse (2) eine zentrale Öffnung (23) besitzt, und zusätzlich ein Mitnehmerelement (3) vorgesehen ist, und das Mitnehmerelement (3) einen Kopf (35) und darin eine Werkzeugansatzstelle (36) besitzt und das Mitnehmerelement (3) einen länglichen Schaft (31) aufweist und dieser Schaft (31) in der Kanülierung (18) des Schafts (13) und in der Gewindebuchse (2, 23) gelagert ist, und wobei das Fenster (17) mit der Kanülierungsöffnung (18) kommuniziert, und der Kopfbereich (11) eine Öffnung (112) mit einem Öffnungsdurchmesser (113) besitzt , welche ebenfalls mit der Kanülierungsöffnung (18) kommuniziert, wobei der Durchmesser der Kanülierungsöffnung (183) kleiner ist als die Öffnungsbreite des Fensters (171) und der Durchmesser der Kanülierungsöffnung (183) ebenfalls kleiner ist als der Öffnungsdurchmesser am Kopf (113) und der Schaft (13) mit dem Klingenbereich (14), dem Halsbereich (12) und dem Kopf (11) mit Kugelsegment (111) insgesamt einstückig ausgebildet ist, **dadurch gekennzeichnet, dass** die Öffnungsbreite (171) des Fensters (17) mindestens gleich groß oder größer ist als der Öffnungsdurchmesser (113) am Kopf

2. Osteosynthesevorrichtung (10) nach einem der vorhergehenden Ansprüche, **dadurch gekennzeichnet, dass** die Flügel (15, 16) innerhalb des Klingenbereichs (14) abschnittsweise mindestens eine Verdickung (155, 165) besitzen, die hauptsächlich parallel zur Zentralachse (103) verläuft, und zur Erhöhung der Biegesteifigkeit der Osteosynthesevorrichtung (10) beiträgt.

3. Osteosynthesevorrichtung (10) nach einem der vorhergehenden Ansprüche, **dadurch gekennzeichnet, dass** diese Verdickungen (155, 165) vorzugsweise an der Außenkante (154, 164) der Flügel (15, 16) lokalisiert sind, und eine größere Dicke (157) als der Flächenabstand der Flügelflächen (153) aufweisen.

4. Osteosynthesevorrichtung (10) nach einem der vorhergehenden Ansprüche, **dadurch gekennzeichnet, dass** die Dicke (157) der Verdickungen (155, 165) mindestens abschnittsweise entlang der Zentralachse (103) variiert.

5. Osteosynthesevorrichtung (10) nach einem der vorhergehenden Ansprüche, **dadurch gekennzeichnet, dass** innerhalb des Klingenbereichs (14) der Kerndurchmesser (131) des Schafts (13) im proximalen Klingenbereich (134) zunimmt.

6. Osteosynthesevorrichtung (10) nach einem der vorhergehenden Ansprüche, **dadurch gekennzeichnet, dass** die Klingenbreite (1516) im proximalen Klingenbereich (144) abnimmt und zum Halsbereich (12) in den Kerndurchmesser des Schafts mündet.

7. Osteosynthesevorrichtung (10) nach einem der vorhergehenden Ansprüche, **dadurch gekennzeichnet, dass** das Fenster (17) innerhalb des Klingenbereichs positioniert ist und sich näher zum proximalen Ende (144) des Klingenbereichs als zum distalen Ende (141) des Klingenbereichs (102) befindet.

8. Osteosynthesevorrichtung (10) nach einem der vorhergehenden Ansprüche, **dadurch gekennzeichnet, dass** die Gewindebuchse (2) eine distale Fläche (22) besitzt und diese distale Fläche (22) in direktem Kontakt mit einer Anlagefläche (172) der Fensterung (17) steht und dieser Kontakt als Anschlag dient.

9. Osteosynthesevorrichtung (10) nach einem der vorhergehenden Ansprüche, **dadurch gekennzeichnet, dass** die Gewindebuchse (2) einen Gewindekerndurchmesser (251) aufweist, welcher in etwa dem Außendurchmesser des Schafts (131) entspricht.

10. Osteosynthesevorrichtung (10) nach einem der vorhergehenden Ansprüche, **dadurch gekennzeichnet, dass** das Mitnehmerelement (3) am Schaftbereich (31) abschnittsweise mindestens ein Profil (34) aufweist und die Gewindebuchse (2) abschnittsweise wenigstens ein dazu kongruentes Profil (24) aufweist, welche miteinander im Eingriff stehen und dazu geeignet sind, ein Drehmoment von der Werkzeugansatzstelle (36) auf die Gewindebuchse zu übertragen.

11. Osteosynthesevorrichtung (10) nach einem der vorhergehenden Ansprüche, **dadurch gekennzeichnet, dass** der Schaft (31) des Mitnehmerelements nach distal (102) aus der Gewindebuchse (2) herausragt und dieses herausragende distale Schaftende in der Kanülierung (18) des Schafts im Klingenbereich (14) gelagert ist, und der Schaft (31) des Mitnehmerelements nach proximal (101) aus der Gewindebuchse (2) herausragt und dieser proximale Schaftabschnitt ebenfalls in der Kanülierung (18) des Schafts (13) gelagert ist.

12. Osteosynthesevorrichtung (10) nach einem der vorhergehenden Ansprüche, **dadurch gekennzeichnet, dass** der Klingenbereich (14), mit den beiden Flügeln (15, 16) eine Breite (1516) zwischen den Außenkanten (154, 164) der Flügel definiert, und ein Gewindeaußendurchmesser (252) der Gewindebuchse (2) definiert ist, wobei der Formfaktor, aus dem Verhältnis aus Breite zwischen den Außenkanten (1516)/Gewindeaußendurchmesser (252), zwischen 1,3 bis 2,5, vorzugsweise 1,4 bis 2,2, vorzugsweise 1,6 bis 2,0 beträgt.

13. System aus mindestens zwei Osteosynthesevorrichtungen (9, 10) nach einem der vorhergehenden Ansprüche, **dadurch gekennzeichnet, dass** mindestens eine der Osteosynthesevorrichtungen (9) eine Verschränkung der Flügelausrichtungen im Uhrzeigersinn (93) und mindestens eine weitere Osteosynthesevorrichtung (10) eine Verschränkung der Flügelausrichtungen entgegen dem Uhrzeigersinn (94) aufweist.

14. System nach dem vorhergehenden Anspruch 13, **dadurch gekennzeichnet, dass** die Osteosynthesevorrichtung eine Markierung oder Beschriftung besitzt, die einen Hinweis auf die Richtung der Verschränkung oder einen Hinweis zur anatomischen Platzierung (91, 92) bereitstellt.

## Claims

1. Osteosynthesis device (10) for the fixation of bone components and bone fragments, in particular vertebrae, comprising a bone anchor (1), wherein the bone anchor (1) has a shaft (13) which extends along a central axis (103) and thereby defines a distal (102) and a proximal (101) direction, and the shaft (13) has at least one blade area (14) with a first (15) and a second wing (16), and proximally (101) adjoins a neck area (12) and further has a head (11) with at least one spherical segment (111), and the bone anchor (1) has a central and continuous cannula opening (18) with a diameter (183), and in the blade area (14) at least one window (17) with an opening width (171) is provided, wherein (17) a threaded bush (2) with a bone thread (25) is rotatably supported in the window and the threaded bush (2) has a central opening (23) and a coupling element (3) is additionally provided and the coupling element (3) has a head (35) and a tool attachement point (36) and the coupling element (3) has an elongated shaft (31) and this shaft (31) is supported in the cannulation (18) of the shaft (13) and in the threaded bush (2, 23) and wherein the window (17) communicates with the cannula opening (18), and the head area (11) has an opening (112) with an opening diameter (113), which also communicates with the cannula opening (18), wherein the diameter of the cannula opening (183) is smaller than the opening width of the window (171) and the diameter of the cannula opening (183) is also smaller than the opening diameter at the head (113) and the shaft (13) is formed integrally with the blade area (14), the neck area (12) and the head (11) with spherical segment (111), **characterized in that**
the opening width of the window (171) is at least equal to or greater than the opening diameter (113) at the head.

2. Osteosynthesis device (10) according to any of the preceding claims, **characterized in that** within the blade area (14) the wings (15, 16) have in sections at least one thickening (155, 165) which runs mainly parallel to the central axis (103) and increases the bending stiffness of the osteosynthesis device (10).

3. Osteosynthesis device (10) according to any of the preceding claims, **characterized in that** said thickenings (155, 165) are preferably located at the top edge (154, 164) of the wings (15, 16) and have a greater thickness (157) than the area distance of the wing areas (153).

4. Osteosynthesis device (10) according to any of the preceding claims, **characterized in that** the thickness (157) of the thickenings (155, 165) varies along the central axis (103) at least in sections.

5. Osteosynthesis device (10) according to any of the preceding claims, **characterized in that** within the blade area (14) the core diameter (131) of the shaft (13) increases in the proximal blade area (134).

6. Osteosynthesis device (10) according to any of the preceding claims, **characterized in that** the blade width (1516) decreases in the proximal blade area (144) and ends at the core diameter of the shaft towards the neck area (12).

7. Osteosynthesis device (10) according to any of the preceding claims, **characterized in that** the window (17) is positioned within the blade area and is closer to the proximal end (144) of the blade area than to the distal end (141) of the blade area (102).

8. osteosynthesis device (10) according to any of the preceding claims, **characterized in that** the threaded bush (2) has a distal area (22) and this distal area (22) is in direct contact with a contact surface (172) of the window (17) and this contact serves as a stop.

9. Osteosynthesis device (10) according to any of the preceding claims, **characterized in that** the threaded bush (2) has a thread core diameter (251) which is approximately equal to the outer diameter of the shaft (131).

10. Osteosynthesis device (10) according to any of the preceding claims, **characterized in that** the coupling element (3) at the shaft area (31) has in sections at least one profile (34) and the threaded bush (2) has in sections at least one profile (24) congruent therewith, which are in engagement with one another and are suitable for transmitting a torque from the tool attachment point (36) to the threaded bush.

11. Osteosynthesis device (10) according to any of the preceding claims, **characterized in that** the shaft (31) of the coupling element protrudes distally (102) from the threaded bush (2) and and this protruding distal shaft end is located in the cannulation (18) of the shaft in the blade area (14), and the shaft (31) of the coupling element protrudes proximally (101) out of the threaded bush (2) and this proximal shaft section is also located in the cannulation (18) of the shaft (13).

12. Osteosynthesis device (10) according to any of the preceding claims, **characterized in that** the blade area (14), with the two wings (15, 16) defines a width (1516) between the top edges (154, 164) of the wings, and a outer thread-diameter (252) of the threaded bush (2) is defined, wherein the form factor, from the ratio of width between the two wings (1516)/ outer thread-diameter (252), is between 1.3 to 2.5, preferably 1.4 to 2.2, preferably 1.6 to 2.0.

13. System of at least two osteosynthesis devices (9, 10) according to any of the preceding claims, **characterized in that** at least one of the osteosynthesis devices (9) has a clockwise (93) twist of the wing orientations and at least one further osteosynthesis device (10) has a counterclockwise (94) twist of the wing orientations.

14. System according to preceeding claim 13, **characterized in that** the osteosynthesis device has a marking or labelling that provides an indication of the direction of the twist or an indication of the anatomical placement (91, 92).

## Revendications

1. Dispositif d'ostéosynthèse (10) pour la fixation de composants osseux et de fragments osseux, en particulier de vertèbres, constitué d'un ancrage osseux (1), dans lequel l'ancrage osseux (1) possède une tige (13) qui s'étend le long d'un axe central (103) et définit ainsi une direction distale (102) et une direction proximale (101), et la tige (13) possède au moins une zone de lame (14) avec une première (15) et une seconde aile (16), et est adjacente de manière proximale (101) à une zone de cou (12) et possède ensuite une tête (11) avec au moins un segment sphérique (111), et l'ancrage osseux (1) présente un orifice de canulation central et continu (18) d'un diamètre (183), et dans la zone de lame (14) est prévue au moins une fenêtre (17) avec une largeur d'orifice (171), dans la fenêtre (17), la douille filetée (2) étant montée mobile en rotation avec un filetage osseux (25) et la douille filetée (2) ayant un orifice central (23) et un élément d'entraînement (3) étant également prévu, et l'élément d'entraînement (3) possède une tête (35) et à son intérieur un emplacement d'insertion d'outil (36) et l'élément d'entraînement (3) a une tige allongée (31) et cette tige (31) est logée dans la canulation (18) de la tige (13) et dans la douille filetée (2, 23), la fenêtre (17) communiquant avec l'orifice de canulation (18), et la zone de tête (11) a un orifice (112) avec un diamètre d'orifice (113), qui communique également avec l'orifice de canulation (18), dans lequel le diamètre de l'orifice de canulation (183) est inférieur à la largeur d'orifice de la fenêtre (171) et le diamètre de l'orifice de canulation (183) est également inférieur au diamètre d'orifice de la tête (113) et la tige (13) avec la zone de lame (14), la zone du cou (12) et la tête (11) est formée dans l'ensemble en un seul tenant avec le segment sphérique (111), **caractérisé en ce que** la largeur d'orifice (171) de la fenêtre (17) est au moins égale ou supérieure au diamètre d'orifice (113) de la tête.

2. Dispositif d'ostéosynthèse (10) selon l'une quelconque des revendications précédentes, **caractérisé en ce que** les ailes (15, 16) possèdent, à l'intérieur de la zone de lame (14), au moins un épaississement (155, 165) par sections, qui s'étend principalement parallèlement à l'axe central (103) et contribue à augmenter la rigidité en flexion du dispositif d'ostéosynthèse (10).

3. Dispositif d'ostéosynthèse (10) selon l'une quelconque des revendications précédentes, **caractérisé en ce que** ces épaississements (155, 165) sont de préférence localisés sur le bord extérieur (154, 164) des ailes (15, 16) et présentent une épaisseur (157) supérieure à l'espacement des surfaces des ailes (153).

4. Dispositif d'ostéosynthèse (10) selon l'une quelconque des revendications précédentes, **caractérisé en ce que** l'épaisseur (157) des épaississements (155, 165) varie au moins par sections le long de l'axe central (103).

5. Dispositif d'ostéosynthèse (10) selon l'une quelconque des revendications précédentes, **caractérisé en ce que** le diamètre du noyau (131) de la tige (13) dans la zone de lame proximale (134) augmente à l'intérieur de la zone de lame (14).

6. Dispositif d'ostéosynthèse (10) selon l'une quelconque des revendications précédentes, **caractérisé en ce que** la largeur de la lame (1516) diminue dans la zone proximale de la lame (144) et débouche vers la zone de cou (12) dans le diamètre du noyau de la tige.

7. Dispositif d'ostéosynthèse (10) selon l'une quelconque des revendications précédentes, **caractérisé en ce que** la fenêtre (17) est positionnée à l'intérieur de la zone de lame et est plus proche de l'extrémité proximale (144) de la zone de lame que de l'extrémité distale (141) de la zone de lame (102).

8. Dispositif d'ostéosynthèse (10) selon l'une quelconque des revendications précédentes, **caractérisé en ce que** la douille filetée (2) possède une surface distale (22) et cette surface distale (22) est en contact direct avec une surface d'appui (172) de la fenêtre (17) et ce contact sert de butée.

9. Dispositif d'ostéosynthèse (10) selon l'une quelconque des revendications précédentes, **caractérisé en ce que** la douille filetée (2) présente un diamètre de noyau fileté (251) qui correspond approximativement au diamètre extérieur de la tige (131).

10. Dispositif d'ostéosynthèse (10) selon l'une quelconque des revendications précédentes, **caractérisé en ce que** l'élément d'entraînement (3) présente, sur la zone de la tige (31), par sections, au moins un profil (34) et la douille filetée (2) présente, par sections, au moins un profil (24) congruent à celui-ci, qui sont en prise l'un avec l'autre et sont adaptés pour transmettre un couple de rotation de l'emplacement d'insertion d'outil (36) à la douille filetée.

11. Dispositif d'ostéosynthèse (10) selon l'une quelconque des revendications précédentes, **caractérisé en ce que** la tige (31) de l'élément d'entraînement fait saillie vers le côté distal (102) de la douille filetée (2) et cette extrémité de tige distale saillante est logée dans la canulation (18) de la tige dans la zone de lame (14), et la tige (31) de l'élément d'entraînement fait saillie vers le côté proximal (101) de la douille filetée (2) et cette partie proximale de la tige est également logée dans la canulation (18) de la tige (13).

12. Dispositif d'ostéosynthèse (10) selon l'une quelconque des revendications précédentes, **caractérisé en ce que** la zone de lame (14), avec les deux ailes (15, 16), définit une largeur (1516) entre les bords extérieurs (154, 164) des ailes, et un diamètre extérieur fileté (252) de la douille filetée (2), le facteur de forme, à partir du rapport de la largeur entre les bords extérieurs (1516)/diamètre extérieur fileté (252), étant compris entre 1,3 et 2,5, de préférence entre 1,4 et 2,2, de préférence entre 1,6 et 2,0.

13. Système d'au moins deux dispositifs d'ostéosynthèse (9, 10) selon l'une quelconque des revendications précédentes, **caractérisé en ce qu'**au moins les dispositifs d'ostéosynthèse (9) présentent un enchevêtrement des orientations des ailes dans le sens des aiguilles d'une montre (93) et au moins un autre dispositif d'ostéosynthèse (10) présente un enchevêtrement des orientations des ailes dans le sens inverse des aiguilles d'une montre (94).

14. Système selon la revendication précédente 13, **caractérisé en ce que** le dispositif d'ostéosynthèse possède un marquage ou une inscription qui fournit une indication de la direction de l'enchevêtrement ou une indication du placement anatomique (91, 92).
